# EUROPEAN PATENT APPLICATION

(11) **EP 1 288 195 A1**
(43) Date of publication of application: **05.03.2003**
(21) Application number: 01307504.9
(22) Date of filing: 04.09.2001
(51) Int. Cl.: C07C 255/27, C07D 211/46, C11D 3/39

(54) **Cationic nitrile compounds and compositions containing them**

(71) Applicant: UNILEVER PLC, London EC4P 4BQ (GB)
(72) Inventor: Rogers, Nicola Jane, Leichhardt, NSW 2040 (AU); Thompson, Katherine Mary, Unilever Res.Port, Wirral, Merseyside, CH63 3JW (GB); Thornthwaite, David William, Unilever Res. Port, Wirral, Merseyside, CH63 3JW (GB)
(74) Representative: Griffiths, Helen Sarah

(57) **Abstract**

The invention provides a cationic ketonitrile containing at least one nitrile group bonded to a quaternary nitrogen atom via an alkylene or substituted alkylene linkage, and at least one ketone grouping bonded to the quaternary nitrogen atom via an alkylene, or substituted alkylene, linkage.

The compounds of the invention are particularly useful as potentiators of the antibacterial activity of peroxygen sources such as hydrogen peroxide, in compositions for the hygienic cleaning of surfaces.

## Description

### Field of the Invention

The present invention generally relates to cationic nitrile compounds which incorporate a ketone grouping (referred to herein as "cationic ketonitriles") and in particular their use in compositions which are designed for the hygienic cleaning of hard surfaces.

### Background and Prior Art

Cleaning compositions designed for application to hard surfaces in the household, or in institutional or hospital environments, generally comprise one or more surfactants, and, optionally, one or more antimicrobial actives and/or solvents.

Hard surfaces found in the household, or in institutional or hospital environments, such as kitchen work surfaces and bathrooms, are often contaminated with bacteria and other microorganisms, which present a risk to human health, especially when they are present near food.

The biocidal activity of surfactants is, with a few notable exceptions, low and it is therefore commonplace to add a separate antimicrobial active to compositions.

Hydrogen peroxide is known as an environmentally friendly antimicrobial oxidant, but typically requires long contact times and/or high concentrations in order to be effective.

The present invention provides new cationic nitrile compounds which have particular utility in hygienic cleaning compositions. The new cationic nitrile compounds have been found to potentiate the antibacterial activity of peroxygen sources such as hydrogen peroxide. This allows a substantial reduction in the concentration of peroxygen source required to achieve good disinfectancy against the typical bacteria as found on hard surfaces in the household, so that effective hygiene performance can be obtained at much lower levels of hydrogen peroxide than would typically be used in a hygiene product such as an antimicrobial household cleaner.

Cationic nitriles of various types have been described as bleach activators for oxygen-containing bleaching agents. Examples are WO96/40661, WO98/23533 and WO98/23534 which all relate to a class of N-alkyl morpholinium acetonitrile compounds which are used in particular to enhance the bleaching activity of peroxygen compounds for laundry bleaching, brightening and stain removal.

EP 0 303 520 describes three particular classes of compound having 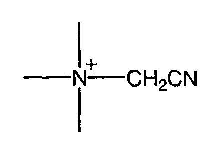 groups in the molecule. These are prepared by reacting a halogenated nitrile with either trialkylamine, pyridine or substituted pyridine, alkylene diamine or polyalkylenepolyamine respectively. The compounds are used as bleach activators for oxygen-containing bleaching agents such as sodium percarbonate and sodium perborate.

EP 0 458 396 and EP 0 464 880 respectively describe how the hygroscopic properties of cationic nitriles of the types disclosed in EP 0 303 520 (in particular those derived from trialkylamines such as trimethylamine) can be improved by either substitution at the α-carbon atom of the cationic nitrile group or by providing certain selected counter-anions.

US 4,874,785 describes a series of bacteriostatic "quats" which contain two long chain alkyl groups, one short chain alkyl group and a functional alkyl group which may be substituted with nitrile ("nitrile quats"). These are said to have a favourable foam profile which allows them to be used without co-additives in formulations used as toilet bowl cleaners and swimming pool algicide.

The present invention provides new cationic nitrile compounds which have particular utility in hygienic cleaning compositions. The new cationic nitrile compounds have been found to potentiate the antibacterial activity of peroxygen sources such as hydrogen peroxide. This allows a substantial reduction in the concentration of peroxygen source (such as H₂O₂) required to achieve good disinfectancy against the typical (Gram + ve & Gram-ve) bacteria as found on hard surfaces in the household.

### Summary of the Invention

The present invention provides a cationic ketonitrile containing at least one nitrile group bonded to a quaternary nitrogen atom via an alkylene or substituted alkylene linkage, and at least one ketone grouping bonded to the quaternary nitrogen atom via an alkylene, or substituted alkylene, linkage.

### Detailed Description and Preferred Embodiments

### Compounds of the Invention

Compounds of the invention may suitably be represented by the general formula (I): 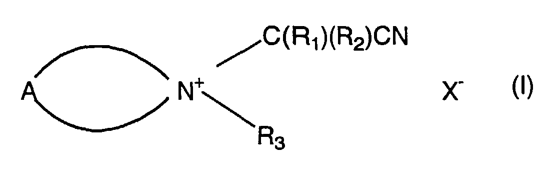 in which A is a carbon chain incorporating at least one carbonylated carbon atom and which, together with the quaternary nitrogen atom, forms a saturated ring system; R₁ and R₂ are each independently selected from hydrogen or a substituent group containing at least one carbon atom; R₃ is selected from C₁₋₁₀ straight or branched chain alkyl (or alkoxylated alkyl where the alkoxy is C₂₋₄), C₇₋₁₂ phenyl-substituted alkyl and C₆₋₁₀ alkyl substituted phenyl; or alternatively R₃ is a substituent group containing at least one carbon atom which is linked to carbon chain A forming a ring; and X is a suitable counterion.

Suitable counterions X include any organic or inorganic anion. Examples are methylsulphate, sulphate, halide (such as chloride, bromide), nitrate, and those counterions described in EP 0 464 880 such as alkane and paraffin sulphonates, p-toluene sulphonate (tosylate), dodecyl benzene sulphonate, primary alcohol sulphates and ether sulphates, alkyl carboxylates and other surfactant anions.

Alternatively, compounds of the invention may suitably be represented by the general formula (II): 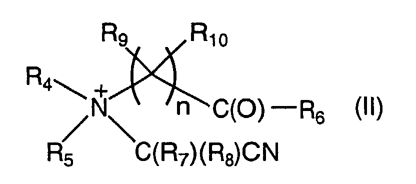 in which R₇ and R₈ are each independently selected from hydrogen or a substituent group containing at least one carbon atom; R₉ and R₁₀ are each independently selected from hydrogen or a substituent group containing at least one carbon atom, which substituent groups R₉ and R₁₀ may optionally be linked to form a ring; R₄ and R₅ are each independently selected from C₁₋₁₀ straight or branched chain alkyl (or alkoxylated alkyl where the alkoxy is C₂₋₄), C₇₋₁₂ phenyl-substituted alkyl and C₆₋₁₀ alkyl substituted phenyl; R₆ is selected from aryl and C₁₋₄ straight or branched chain alkyl (or alkoxylated alkyl where the alkoxy is C₂₋₄); n is an integer from 1 to 4 and X is a suitable counterion such as is described above.

### Compositions

The novel compounds of the present invention are particularly useful as potentiators of the antibacterial activity of peroxygen sources such as hydrogen peroxide.

Accordingly the present invention also provides a composition suitable for the hygienic cleaning of surfaces comprising a compound of the invention as described above (hereinafter referred to as a "cationic ketonitrile"), together with a peroxygen source.

### Cationic Ketonitrile

Compositions of the invention comprise one or more cationic ketonitriles as are described above.

Preferred compounds of general formula (I) for inclusion in compositions according to the invention include those in which carbon chain A has one of the formulae below: 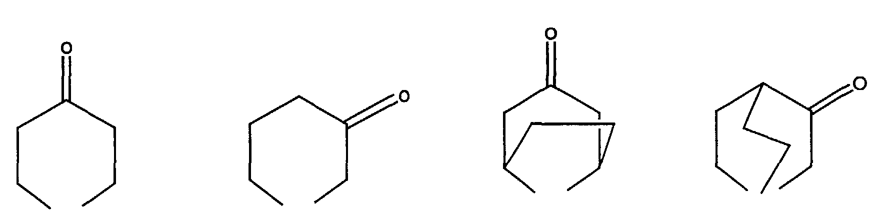 R₁ and R₂ are both hydrogen and R₃ is methyl or ethyl.

Especially preferred are compounds of general formula (I) in which A has the formula: 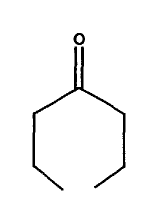 R₁ and R₂ are both hydrogen and R₃ is methyl. These may be designated as N-acetonitrile-N-methyl-4-oxo-piperidinium salts.

Preferred compounds of general formula (II) for inclusion in compositions according to the invention include those in which R₇, R₈, R₉ and R₁₀ are all hydrogen, R₄ and R₅ are both ethyl and R₆ is methyl.

In compositions of the invention, the total amount of cationic ketonitrile suitably ranges from 0.01% to 10%, more preferably from 0.1% to 5%, most preferably from 0.15% to 4.0% by weight based on total weight of the composition.

### Peroxygen Source

The peroxygen source may be any peroxide or peroxide generating system known in the art such as dihydrocarbon peroxides, diacyl peroxides, hydrocarbon hydroperoxides, organic and inorganic peracids and persalts and in particular hydrogen peroxide.

Preferred peroxygen sources are hydrogen peroxide, peracetic acid, PAP and alkali metal or alkaline earth metal monoperoxosulphate salts.

Hydrogen peroxide is most preferred and may be incorporated *per se* into the composition, or may be generated *in situ* by use of a hydrogen peroxide precursor. Examples of suitable hydrogen peroxide precursors are compounds which produce hydrogen peroxide on dissolution in water, (such as sodium perborate monohydrate, sodium perborate tetrahydrate, sodium percarbonate and the urea-hydrogen peroxide addition compound percarbamide), and enzymatic hydrogen peroxide generating systems (such as peroxidases, oxidases and other oxido-reductase enzyme systems, in conjunction with their appropriate substrates).

Advantageously the cationic ketonitrile potentiates the antibacterial activity of the peroxygen source. This allows effective hygiene performance to be obtained at much lower levels of active than would typically be used in a hygiene product such as an antimicrobial household cleaner.

The benefits of being able to formulate at reduced levels of active are particularly significant in the context of household cleaning. Such advantages include the reduction or elimination of potential skin or eye irritation associated with consumer usage of the formulation, and the reduction or elimination of formulation residues which could, for example, contaminate food.

The amount of hydrogen peroxide in compositions of the invention (or precursor thereof generating the equivalent amount of hydrogen peroxide), suitably ranges from 0.0001% to 5%, more preferably from 0.001 to 1.5%, most preferably from 0.003% to 0.6%, by weight based on total weight of the composition.

### Ingredient Ratios

The molar ratio of cationic ketonitrile (in total, if a mixture of cationic ketonitriles is used) to peroxygen source (such as hydrogen peroxide or precursor thereof generating the equivalent amount of hydrogen peroxide) typically ranges from 10:1 to 1:1000, preferably from 5:1 to 1:100, most preferably about 1:1.

### pH

Aqueous ready-to-use compositions of the invention will generally have a pH between 6 and 12, more preferably between 7 and 11.5.

### Product Usage

Compositions according to the invention are designed in particular for the hygienic cleaning of hard surfaces. By "hard surfaces" is meant those surfaces which are typically found in the household, or in institutional or hospital environments, and which are prone to microbial contamination. Examples include bathroom fixtures, bathroom appliances (toilets, bidets, shower stalls, bathtubs and bathing appliances), wall and flooring surfaces and those surfaces associated with kitchen environments and other environments associated with food preparation.

### Product Form and Packaging

Compositions of the invention designed for the hygienic cleaning of surfaces may take any form suitable for hygiene products.

Liquid compositions according to the invention are typically aqueous compositions in which the principal ingredient is water, which is normally present at a level of at least 75%, preferably at least 90%, more preferably at least 95%, by weight based on total weight of the composition. The use of distilled or demineralised water is preferred, but not essential to the invention.

In liquid compositions according to the invention, it is preferred to store the cationic ketonitrile separately from the peroxygen source.

This could be achieved by providing them in respective separate containers. The consumer could then apply each to the surface to be treated, either sequentially or simultaneously. However, it is more convenient to package the composition in a dual-compartment container in which the cationic ketonitrile and peroxygen source are stored in separate compartments. Such a dual-compartment container will include a delivery means which allows the cationic ketonitrile and peroxygen source to be delivered to the surface to be treated so that they combine as they are exiting the delivery means and/or in mid-air as they are directed to the surface and/or on the surface itself.

A particularly preferred delivery means, especially for non-thickened systems, is a trigger spray head. In the case of a dual compartment system, this will preferably have two siphon tubes, respectively leading into each compartment and either a single nozzle with a mixing chamber or two separate nozzles substantially adjacent to each other. If desired, a dispensing nozzle or nozzles configured to promote foaming may be used.

For thickened systems a pouring dual compartment packaging form is generally preferred.

Alternatively, compositions of the invention may contain a peroxygen source precursor such as a hydrogen peroxide precursor which produces hydrogen peroxide on dissolution in water, in which case a solid product form can be used, such as a tablet.

### Other Ingredients

Compositions of the invention designed for the hygienic cleaning of surfaces may further contain any conventional surfactant for emulsification and cleaning purposes. The concentration of this surfactant material is generally in the range from 0 to 50% by weight based on total weight of the composition, but a maximum concentration of 20% by weight based on total weight of the composition is usually preferred. Suitable surfactants may be anionic surfactants, such as alkyl aryl sulfonates, alkyl sulfates and alkyl sulfonates, nonionic surfactants, such as ethylene oxide and/or propylene oxide condensation products with alcohols or alkylphenol, or mixtures thereof.

The composition according to the invention can contain other optional ingredients which aid in their cleaning performance and maintain the physical and chemical stability of the product.

Hydrotropes are useful optional components. These enable the cloud point of the composition to be raised without requiring the addition of anionic surfactants.

Suitable hydrotropes include alkali metal toluene sulphonates, urea, alkali metal xylene and cumene sulphonates, short chain (preferably C₁₋₅) alcohols, and glycols. Preferred amongst these hydrotropes are the sulphonates, particularly the cumene and toluene sulphonates.

Typical levels of hydrotrope range from 0 to 5% by weight based on total weight of the composition, for the sulphonates. Correspondingly higher levels of urea and alcohols are required. Hydrotropes are not required for dilute products.

Solvents may be present in the compositions of the invention.

Suitable solvents correspond to the general formula R₁-O-(EO)ₘ-(PO)ₙ-R₂, wherein R₁ and R₂ are independently C₂₋₆ alkyl or hydrogen, but not both hydrogen, m and n are independently 0-5, EO represents an ethyleneoxy group and PO represents a propyleneoxy group.

Suitable materials of this type include the C₁₋₄ alkyl ethers of alkylene glycols such as ethylene glycol, propylene glycol and oligomers thereof having 2 or 3 repeating units. Examples include monoethers such as ethylene glycol mono-n-butyl ether, ethylene glycol monomethyl ether, ethylene glycol monoethyl ether, ethylene glycol mono-n-hexyl ether, propylene glycol monomethyl ether, propylene glycol monoethyl ether, propylene glycol mono-n-butyl ether, isopropylene glycol monoethyl or monopropyl or monobutyl ether, diethylene glycol monoethyl or monopropyl or monobutyl ether, di- or tripropylene glycol monomethyl ether, di- or tripropylene glycol monoethyl ether, and mixtures thereof.

Typical levels of solvent range from 0 to 2% by weight based on total weight of the composition.

Compositions according to the invention can also contain, in addition to the ingredients already mentioned, other optional ingredients such as pH regulants, sunscreens, foam-control agents, colorants, viscosity modifying agents, freeze-thaw stabilisers, additional hygiene agents, perfumes and opacifiers.

The invention will now be illustrated by the following nonlimiting Examples, in which all percentages are by weight based on total weight, unless otherwise indicated.

### EXAMPLES

### Example 1: Bactericidal activity

The bactericidal activities of various combinations of hydrogen peroxide and a representative cationic ketonitrile, compared with controls using either ingredient individually, were tested using the European Suspension Test protocol (European standard EN1276). Biocidal test data against two representative bacteria, *E. coli* ATCC 11229 (Gram -negative) and *S. aureus ATCC 6538* (Gram-positive) are shown below in Table 1.

**Table 1 (a):**

| Bactericidal activity from combinations of N-acetonitrile-N-methyl-4-oxo-piperidinium bromide and hydrogen peroxide (5 minutes contact time) in unbuffered systems against *E. coli*: | | | |
|---|---|---|---|
| | | **Mean log reduction in cell numbers**^{**(1)**} **(from log-7 cell suspension)** | |
| **Formulation** | **pH** | No BSA⁽²⁾ | 0.3% BSA |
| 0.1% hydrogen peroxide | 11.0 | 0 | 0 |
| 0.7% N-acetonitrile-N-methyl-4-oxo-piperidinium bromide | 11.0 | 0 | 0 |
| 0.1% hydrogen peroxide + 0.7% N-acetonitrile-N-methyl-4-oxo-piperidinium bromide | 11.0 | 4.8 | 2.0 |
| 0.1% hydrogen peroxide | 9.2 | 0 | 0 |
| 0.7% N-acetonitrile-N-methyl-4-oxo-piperidinium bromide | 9.2 | 0 | 0 |
| 0.1% hydrogen peroxide + 0.7% N-acetonitrile-N-methyl-4-oxo-piperidinium bromide | 9.2 | 4.7 | 3.5 |
| 0.1% hydrogen peroxide | 7.0 | 0 | 0 |
| 0.7% N-acetonitrile-N-methyl-4-oxo-piperidinium bromide | 7.0 | 0 | 0 |
| 0.1% hydrogen peroxide + 0.7% N-acetonitrile-N-methyl-4-oxo-piperidinium bromide | 7.0 | 2.9 | 1.0 |

| | | | |
|---|---|---|---|
| (1) 4 replicate experiments | | | |
| (2) BSA - bovine serum albumin (protein soil) | | | |

**Table 1 (b):**

| Bactericidal activity from combinations of N-acetonitrile-N-methyl-4-oxo-piperidinium bromide and hydrogen peroxide (5 minutes contact time) in unbuffered systems against *S. aureus:* | | | |
|---|---|---|---|
| | | **Mean log reduction in cell numbers**^{**(1)**} **(from log-7 cell suspension)** | |
| **Formulation** | **pH** | No BSA⁽²⁾ | 0.3% BSA |
| 0.5% hydrogen peroxide | 11.0 | 0 | 0 |
| 3.5% N-acetonitrile-N-methyl-4-oxo-piperidinium bromide | 11.0 | 0 | 0 |
| 0.5% hydrogen peroxide + 3.5% N-acetonitrile-N-methyl-4-oxo-piperidinium bromide | 11.0 | 5.0 | 5.0 |

| | | | |
|---|---|---|---|
| ⁽¹⁾ 4 replicate experiments | | | |
| ⁽²⁾ BSA - bovine serum albumin (protein soil) | | | |

The data shows that the bactericidal performance of the cationic ketonitrile/hydrogen peroxide combination is significantly greater than that observed from the same levels of either the cationic ketonitrile or hydrogen peroxide alone.

### Example 2: Synthesis of N-Methyl-4-oxo- piperidinium-acetonitrile bromide

N-Methyl-4-oxopiperidine (5.65g, 0.05m) was dissolved in acetonitrile (30ml) and bromoacetonitrile (5.95g, 0.05m) was added. The mixture was heated under reflux for 6h. The solvent was cooled and a white solid crystallised out of solution, was separated by filtration, washed with acetonitrile and dried *in vacuo* 8.4g,72% yield; ¹H NMR : (δ, D₂O) 4.24, m, 4H, O=C(CH₂C**H**_{**2**}**)**_{**2**}-N⁺; 3.75, m, 4H, O=C(C**H**_{**2**}CH₂)₂N⁺; 3.4, s, 3H, **Me**N⁺.

## Claims

1. A cationic ketonitrile containing at least one nitrile group bonded to a quaternary nitrogen atom via an alkylene or substituted alkylene linkage, and at least one ketone grouping bonded to the quaternary nitrogen atom via an alkylene, or substituted alkylene, linkage.

2. A cationic ketonitrile according to claim 1, having the general formula (I): 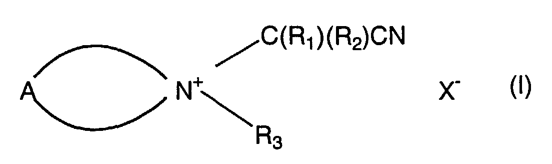 in which A is a carbon chain incorporating at least one carbonylated carbon atom and which, together with the quaternary nitrogen atom, forms a saturated ring system; R₁ and R₂ are each independently selected from hydrogen or a substituent group containing at least one carbon atom; R₃ is selected from C₁₋₁₀ straight or branched chain alkyl (or alkoxylated alkyl where the alkoxy is C₂₋₄), C₇₋₁₂ phenyl-substituted alkyl and C₆₋₁₀ alkyl substituted phenyl; or alternatively R₃ is a substituent group containing at least one carbon atom which is linked to carbon chain A forming a ring; and X is a suitable counterion.

3. A cationic ketonitrile according to claim 1, having the general formula (II): 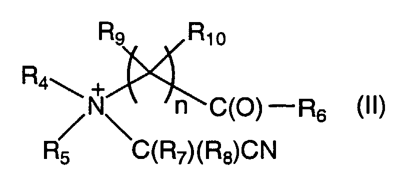 in which R₇ and R₈ are each independently selected from hydrogen or a substituent group containing at least one carbon atom; R₉ and R₁₀ are each independently selected from hydrogen or a substituent group containing at least one carbon atom, which substituent groups R₉ and R₁₀ may optionally be linked to form a ring; R₄ and R₅ are each independently selected from C₁₋₁₀ straight or branched chain alkyl or alkoxylated alkyl where the alkoxy is C₂₋₄, C₇₋₁₂ phenyl-substituted alkyl and C₆₋₁₀ alkyl substituted phenyl; R₆ is selected from aryl and C₁₋₄ straight or branched chain alkyl or alkoxylated alkyl where the alkoxy is C₂₋₄; n is an integer from 1 to 4 and X is a suitable counterion.

4. A cationic ketonitrile of general formula (I) as defined in claim 2, in which carbon chain A has one of the formulae below: 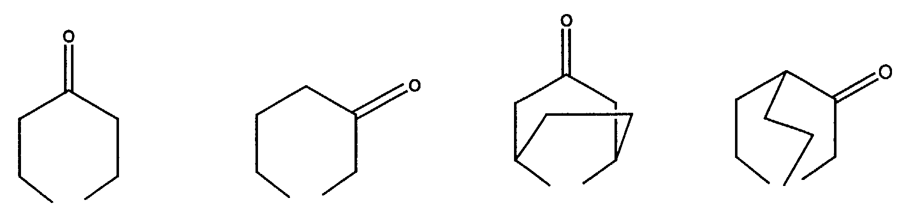 R₁ and R₂ are both hydrogen and R₃ is methyl or ethyl.

5. A cationic ketonitrile of general formula (II) as defined in claim 3, in which R₇, R₈, R₉ and R₁₀ are all hydrogen, R₄ and R₅ are both ethyl and R₆ is methyl.

6. A composition suitable for the hygienic cleaning of surfaces which comprises a cationic ketonitrile as defined in any one of claims 1 to 5, together with a peroxygen source.

7. A composition according to claim 6, in which the peroxygen source is hydrogen peroxide.

8. A composition according to claim 7, in which the molar ratio of cationic ketonitrile to hydrogen peroxide ranges from 10:1 to 1:1000, preferably from 5:1 to 1:100, most preferably about 1:1.

9. A composition according to any one of claims 6 to 8, which has a pH between 6 and 12, preferably from 7.0 to 11.5.

10. A hygienic cleaning composition according to any one of claims 6 to 9, in which the cationic ketonitrile and peroxygen source are respectively packaged in separate compartments of a dual compartment dispenser and mixed on dispensation.
